# FASCICULE DE BREVET EUROPEEN

(11) **EP 1 260 509 B1**
(45) Date de publication et mention de la délivrance du brevet: **21.04.2010**
(21) Numéro de dépôt: 02291281.0
(22) Date de dépôt: 24.05.2002
(51) Int. Cl.: C07D 311/72, A61K 31/355

(54) **Utilisations d'esters de tocophérol**
Anwendungen von Tocopherolestern
Uses of tocopherol esters

(30) Priorité: 25.05.2001 FR 0106897
(43) Date de publication de la demande: 27.11.2002
(73) Titulaire: Derma Developpement S.A.R.L., La Molière, 78290 Croissy sur Seine (FR)
(72) Inventeur: Brin, André Jean, 78290 Croissy sur Seine (FR); Tapiero, Claude, 34080 Montpellier (FR)
(74) Mandataire: Célanie, Christian

(56) Documents cités:
- EP-A- 0 231 777
- EP-A- 0 356 270
- WO-A-00/02554
- WO-A-99/32105
- FR-A- 2 420 523
- DATABASE CA [en ligne] CHEMICAL ABSTRACTS SERVICE, COLUMBUS, OHIO, US; MIYASHITA, SHIGEO ET AL: "Skin cosmetics" retrieved from STN Database accession no. 80:63760 XP002189539 & JP 48 014932 B (POLA CHEMICAL INDUSTRY CO., LTD.) 11 mai 1973 (1973-05-11)
- PATENT ABSTRACTS OF JAPAN vol. 010, no. 203 (C-360), 16 juillet 1986 (1986-07-16) -& JP 61 044853 A (TERUMO CORP), 4 mars 1986 (1986-03-04)
- DATABASE CA [en ligne] CHEMICAL ABSTRACTS SERVICE, COLUMBUS, OHIO, US; MOTOI, TOSHIYUKI: "Hair tonics containing tocopherol linoleate" retrieved from STN Database accession no. 106:162380 XP002189545 & JP 61 289022 A (KANEBO, LTD., JAPAN) 19 décembre 1986 (1986-12-19)
- DATABASE CA [en ligne] CHEMICAL ABSTRACTS SERVICE, COLUMBUS, OHIO, US; SIMIDZHIEV, I. ET AL: "Effect of alpha-tocopherol and its derivatives on ATPase activity and oxidative phosphorylation in rat liver mitochondria" retrieved from STN Database accession no. 108:54735 XP002189547 & BYULL. EKSP. BIOL. MED. (1987), 104(9), 299-301,
- CHEMICAL ABSTRACTS, vol. 54, no. 4, 25 février 1960 (1960-02-25) Columbus, Ohio, US; abstract no. 3401b, P.F.G. PRAILL: XP002190483 & J. CHEM. SOC., 1959, pages 3100-3101,

## Description

La présente invention se rapporte à l'utilisation d'esters de Tocophérol avec des acides gras mono ou polyinsaturés de la série oméga-3 ou de la série oméga-6, répondant à la formule générale I 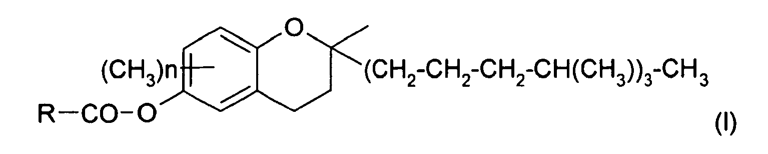 dans laquelle :
- n est un nombre entier variant de 1 à 3
- R est le reste de la chaîne aliphatique d'un acide gras monoinsaturé ayant de 11 à 22 atomes de carbone, ou d'un acide gras polyinsaturé choisi parmi les acides α-linolénique (C18:3 n-3) , octadécatétraénoïque (C18:4 n-3), eicosatétra énoïque (C20:4 n-3), docosahexaénoïque (C22:6 n-3), linoléique (C18:2 n-6), γ-linolénique (C18:3 n-6), dihomo- γ-linolénique (C20:3 n-6), arachidonique (C20:4 n-6), et docosapentaénoïque (C22:5 n-6).

A titre d'exemple d'acides gras mono-insaturés ayant de 1-1 à 22 atomes de carbone, on pourra citer les esters d'acides undécylénique (C11 :1), et les esters d'acides palmitoléique (C16:1)

Parmi les composés de formule générale I on distinguera les esters de tocophéryle et notamment les esters d' α-tocophérol, les esters de β- tocophérol, les esters de γ-tocophérol et les esters de δ-tocophérol. Les tocophérols peuvent être sous forme racémique (*dl*) ou sous forme optiquement active (*d ou l*).

Les composés de formule générale I selon l'invention peuvent être obtenus par un procédé qui consiste à préparer un dérivé fonctionnel d'acide gras mono ou polyinsaturé, puis à le faire réagir, éventuellement en présence d'un activateur du carboxyle et/ ou d'un catalyseur d'estérification avec un tocophérol et à isoler l'ester de tocophérol et d'acide gras mono ou polyinsaturé.

Les composés de formule générale I selon l'invention peuvent être obtenus par un procédé qui consiste à transformer l'acide gras mono ou polyinsaturé en halogénure d'acide. La réaction d'estérification proprement dite est réalisée avec l' α-tocophérol en solution dans la pyridine. On peut activer la réaction en ajoutant une base tertiaire comme la triéthylamine ou la 4-diméthyl aminopyridine. Comme halogénure d'acide gras mono ou poly insaturé on pourra citer le chlorure, le bromure ou l'iodure ; le chlorure est le réactif préféré. On pourra également utiliser un anhydride d'acide ou un anhydride mixte avec un carbodiimide.

La méthode de purification utilisée est la chromatographie liquide sur colonne de gel de silice.

Les esters obtenus dans la présente invention ont été analysés par spectrométrie Infra rouge. Des bandes caractéristiques de ce type d'ester ont été déterminés :
3010 cm⁻¹= alcène ou alcane selon qu' on a une bande intense ou fine
2926 cm⁻¹= alcène ou alcane selon qu' on a une bande intense ou fine
2859 cm⁻¹= alcène ou alcane selon qu' on a une bande intense ou fine
1756 cm⁻¹= carbonyle de la fonction ester (bande intense)
1460/ 1376 cm⁻¹ =CH 3-CH bande intense/ moyenne
1136/ 1109 cm⁻¹ 2 bandes intenses avec une différence de 30 cm-1 = éther/ éther aromatique
920 cm⁻¹= alcène
734 cm⁻¹= alcène

L' invention a également pour objet des compositions pharmaceutiques, cosmétiques et/ou diététiques, ayant une utilité chez l'Homme ou l'animal, et renfermant comme principe actif un ou des esters d'acide gras non saturé de tocophérol de formule générale I avec des excipients ou des véhicules appropriés.

Ces compositions ont pour objet l'utilisation en application topique des esters d'acide gras non saturé de tocophérol.
L'invention peut donc se trouver sous la forme libre ou sous forme encapsulée ou sous forme de liposome pour une meilleure diffusion.

Les compositions de la présente invention sont particulièrement aptes à l'application locale notamment sous la forme de gel, de crème H/E ou E/H, d'émulsion, de lotions, de pommade, de poudre, de lotion capillaire, de spray, d'ampoule, de sérum ou de patches.
Elles sont également adaptées pour l'administration, notamment par voie orale, sous forme de solution buvable ou de capsule.

Dans ces compositions, la teneur en principe actif de formule générale I peut varier de 0,001 % à 15 % et plus particulièrement de 0,01 à 5% en fonction du mode d' administration ou du mode d'application de ces compositions.

Elles renferment en outre des agents solubilisants, des polymères épaississants, des huiles de silicone, des agents stabilisants, des agents émulsionnants.

D'une manière préférée, on utilisera des supports destinés à être dilués ou incorporés dans des préparations telles que des crèmes ou des gels. La phase huileuse contenant éventuellement des agents tensioactifs non ioniques et/ou des silicones est ensuite additionnée d'une phase aqueuse contenant éventuellement un agent gélifiant ou un agent épaississant. Après agitation prolongée, on obtient ainsi une émulsion que l'on incorpore dans une base cosmétique, grasse ou non grasse, pour réaliser une crème ou un gel. On procédera de manière semblable pour les préparations à phase continue huileuse.

La phase huileuse contient éventuellement des agents tensioactifs non ioniques et/ou des silicones.

On pourra également utiliser l'ester d'acide gras de tocophérol dans la glycérine, l'incorporer dans une solution de polyéthylèneglycol contenant un agent dispersant ou tensioactif, et l'additionner d'une huile de silicone pour réaliser une émulsion fluide que l'on peut, si désiré, colorer et/ou parfumer à l'aide d'une essence de fleurs ou de plantes naturelle ou bien encore avec une substance odoriférante comme une ionone le lavendulol ou un parfum.

Les compositions peuvent ainsi contenir un agent émulsionnant comme un stéarate d'éthylène glycol, un stéarate de sorbitol + polysorbate 60, le PEG-20 sesquistéarate de méthyl glucose, le PEG-30 dipoly hydroxy stéarate + sorbitol et esters de glycérol, ou encore le cétyl dimethicone copolyol.

Parmi les polymères que l'on peut adjoindre, on citera en particulier les agents épaississants comme les polymères d'éthylène glycol comme le PEG 400, le PEG 600 ou des dérivés de la cellulose comme la hydroxy éthyl cellulose ou l'hydroxypropyl méthyl cellulose ou la méthyl cellulose.

Parmi les solubilisants , on peut adjoindre à la préparation soit des huiles polaires telles que l'huile de jojoba, des triglycérides d'acide caprylique ou caprique, de I' huile de macadamia, de I' huile de cameline, de I' huile de lin ou de I' huile de kiwi, soit des carbures apolaires comme l'isohexadécane, de l'huile minérale, du squalane soit encore des huiles siliconées comme la diméthicone, la cyclométhicone, la phenyl triméthicone, le mélange de cyclopentasiloxane et de diméthycone copolyol.

Comme gélifiants on peut citer à titre d'exemple les carbomères tels que les acrylates/C10-30 alkoyl acrylate de polymère croisé 1,2 et 3 ou des gels naturels comme la gomme xanthane ou les alginates tels que l'alginate de sodium, un mélange de polyacrylamide et de C13-14 isoparaffine et de laureth-7 polysaccharide.

### ART ANTERIEUR

La littérature fournit un grand nombre de références relatives à des esters de tocophérol et plus particulièrement à des esters d'acide gras et de tocophérol. Les propriétés des produits en découlant sont très variées :
- DCV WO 99/32105 qui devient des esters de tocophérol d'une manière très générale, possédant des propriétés biologiques et notamment celles associées à celles d'un polyène, comme des propriétés antitumorales.
- Nisshin Flour Milling CO (FR 2.420.52) décrit des esters d'acide gras avec le γ - tocophérol. Ces composés ont une action anti-ulcéreuse préventive ou curative.
- Brevet WO 00/02554 (Panin G) décrit en général des esters de tocophérol α dans le traitement de la pathologie de la muqueuse buccale grâce à des propriétés de capteur de radicaux libres.
- Brevet européen 0231 777 (Hoffmann - La Roche) décrit des compositions cosmétiques à base d'un mélange de lino-leate d'α tocophérol et d'un ou plusieurs autres esters d'acide gras et de tocophérol α. Ces compositions manifestent des propriétés de rétention de l'humidité par application topique.
- La publication Smidziev et al (Bull Eksp Biol. Med. (1987) 104 (9) 299.301) décrit l'effet de l'α tocophérol et de ses esters (acétate, stéarate....) sur les mitochondries du foie du rat. Les auteurs ont montré que ces dérivés ont un effet inhibiteur sur la stimulation de l'ATP induite par le 2.4 - dinitrophérol.
- La référence Terumo (JP 61044853) décrit la préparation d'esters d'acide gras avec le tocophérol α. En particulier l'ester d'acide γ linoléique est figuré comme agent préventif ou curatif du cancer. Il supprimerait remarquablement les phénomènes de coagulation des thrombocytes induits par l'acide arachidonique ou par le collagène.
- La référence Pola Chemical Industry CO (Chem Abst. (1974) 63760) décrit des compositions cosmétiques telles que des lotions, des émulsions, des crèmes contenant de 0,1 à 10% en poids d'esters de tocophérol avec l'acide arachidonique, l'acide linoléique ou l'acide linolénique. De tels esters auraient principalement des propriétés anti-inflammatoires.
- La référence Kanebo LTD (Chem - Abst. 106 162 380) décrit des lotions capillaires contenant du linoleate de DLα-tocophérol et d'huile de ricin hydrogéné polyoxy-ethylénique comme agent favorisant la pousse des poils chez la souris.
- La référence EP 0356270 (R-Azar) décrit entre autres des esters d'acide γ linolénique avec un tocophérol. Ces esters sont mentionnés comme produit de cosmétologie.

Les compositions peuvent être employées plus efficacement que les compositions de l'art antérieur qui utilisent une simple association d'acide gras poly-insaturé et d'α tocophérol, notamment en ce qui concerne :
* l'influence sur la synthèse des constituants du derme comme le collagène, l'élastine, l'acide hyaluronique et les protéoglycanes.
* l'influence sur la synthèse des lipides cutanés de type céramides et sur les métallo-protéases et protéines kinases.
* l'action anti-vieillissement
* l'action anti-radicalaire HX/XO et anti UVB.
* l'action anti-inflammatoire
* l'effet stimulant sur la capacité respiratoire des mitochondries.
* l'action énergisante sur les cellules.

### Exemple 1 : exemple de formulations contenant un composé de formule générale I

### Formulation 1 :

### Crème

γ-linolénate d'α tocophéryle 0.5 %
Glycérine 5%
Poly propylène de glycérol-26-butyreth-26 4%
Poly éthylène glycol-40 dans l'huile de ricin hydrogénée 4% eau qsp 100

### Formulation 2 :

### Capsule

y-linolénate d'α tocophéryle 2 %
Huile de cameline 1000 mg
pour une capsule

### Formulation 3 :

### Crème

Alcool cétéarylique et ceteareth-20 3,5 %
Acool cétylique 3,5 %
Palmitate d'octyle 9%
y-linolénate d'α-tocophéryle 0.5 %
Caséine- Gomme xanthane 1%
eau qsp 100

### Formulation 4 :

### Emulsion huile dans l'eau

| | | |
|---|---|---|
| Phase A | Glycérol | 4% |
| | Carbomère | 1 |
| Phase B | Eau désionisée | qsp100 |
| Phase C | PEG-78-glyceryl cocoate γ-linolénate d'α tocophéryle 0.5 % | 1 |
| | Huile essentielle d'orange amère | 0,1 |
| Phase D | Conservateur (s) | 0,7 |
| Phase E | NaOH diluée à 10% | qsp pH=6 |

Additionner la phase A dans la Phase B.
Sous agitation additionner la Phase C.
Puis ajouter la phase C sous forte agitation
Une fois homogénéisation complète, ajouter D.
Stabiliser le pH à 5 -6 par de la soude diluée.

### Exemple II :

### ETUDE PHARMACOLOGIQUE DES COMPOSES DE FORMULE I

### 1. Etude d'un ester de tocophéryle avec un acide gras mono ou polyinsaturé, sur l'activité de la tyrosinase sur des mélanocytes humains.

### 5. Préparation des cellules

Les mélanocytes humains sont ensemencés à la densité de 50 000 cellules/cm² dans des plaques multi puits.
Les cellules sont incubées à 37°C en atmosphère humide contenant 5% (v/v) de CO₂. Le milieu de culture est le MCDB 153 supplémenté par l'EGF (5 ng/mL) (Sigma), l'insuline (5 µg/mL)(Sigma), l'hydrocortisone (5 ng/mL)(Sigma),du sérum de veau foetal (SVF) à 5 % (v/v)( Gibco).

Le milieu de culture est remplacé après 24 heures de culture et supplémenté par des antibiotiques (Peni-Strepto)(Gibco) à 1% (V/v) contenant les différentes concentrations d'un ester de tocophéryle avec un acide gras mono ou polyinsaturé (1, 10 et 100 µg/mL).

Ce sont des concentrations non cytotoxiques qui ne modifient pas la croissance cellulaire.

Une série « témoin positif» est constituée de cellules traitées à l'hydroquinone à 1 µg/mL.

### b) Evaluation de l'activité tyrosine kinase.

Les cellules sont incubées pendant 72 heures à 37°C en atmosphère humide contenant 5% (v/v) de CO₂.
A la fin de la période d'incubation, le milieu de culture est prélevé et la couche cellulaire rincée deux fois avec du PBS. 450 µL de Triton X100 à 1% (v/v) dans le PBS sont ajouté dans chaque puits. On agite pendant 10 minutes puis on ajoute 50 µL de L-Dopamine à 10 mM dans du PBS sans Ca⁺⁺ ni Mg ⁺⁺
On laisse incuber pendant 1 heure à 37°C à l'abri de la lumière, puis on mesure l'absorption à 475 nm au spectrophotomètre.

**Tableau 1**

| | témoin | hydroquinone | 1 µg/mL | 10 µg/mL | 100 µg/mL |
|---|---|---|---|---|---|
| Moyenne absorbance à 475 nm | 0.044 ± 0.002 | 0.024 ± 0.001 | 0.046 ±0.000 | 0.052 ± 0.003 | 0.050 ±0.002 |

### Résultats

Un ester de tocophéryle avec un acide gras mono ou polyinsaturé à la concentration de 10 µg/mL exerce une stimulation de la tyrosinase de l'ordre de 18 %.

### 2. Etude d'un ester de tocophéryle avec un acide gras mono ou polyinsaturé sur l'activité anti-inflammatoire sur une préparation de monocytes/macrophages humains activés ou non par les LPS (lipopolysaccharides).

### a) Préparation des monocouches de monocytes/macrophages humains.

Les monocytes/macrophages sont purifiés à partir d'une poche de sang. Le « Buffy coat » est prélevé stérilement et dilué 7 fois dans un tampon PBS-EDTA.
Une solution de Percoll (Pharmacia) est préparée extemporanément :
- solution A : 9 mL de Percoll + 1 mL de NaCl 1,5 M
- solution B : 6 mL de solution A + 4 mL de NaCl 1,5 M
24 tubes sont préparées ( 7 mL de « Buffy coat » dilué + 4 mL de solution B, centrifugés 800 G pendant 30 minutes à température ambiante.

L'interface contenant les cellules mononuclées est prélevée et rincée 2 fois avec du PBS-EDTA (5mM) puis avec du milieu Ham F12 (Gibco) supplémenté avec du sérum de veau foetal (SVF) à 10 % inactivé par la chaleur.

Le culot est suspendu dans du milieu Ham F12 supplémenté par de la L-glutamine (2mM) et du SVF à 20%. On obtient une suspension à 4 x 10⁶ cellules/mL.

Les cellules sont ensemencées à la densité de 4 x 10⁶ cellules/puits dans des plaques multipuits.

Les cellules sont incubées à 37°C en atmosphère humide contenant 5% (v/v) de CO₂ pendant 2 heures de façon à obtenir une population cellulaire adhérente de 110 000 à 150 000 cellules/cm² composée à 95 % de monocytes/macrophages. Le milieu est ensuite remplacé par du Ham F12 supplémenté par du SVF à 5 % et de la L-glutamine à 2 mM.

Une série « témoin positif» est constituée de cellules traitées au phénol à 640 µg/mL. L'étude est réalisée avec différentes concentrations d'un ester de tocophéryle avec un acide gras mono ou polyinsaturé ( 1, 10, 50,100 et 500 µg/mL).

Deux séries de tubes sont supplémentés par 1 et 5 µg/mL de LPS.

Les monocytes/macrophages sont incubés pendant 24 heures à 37°C en atmosphère humide contenant 5% (v/v) de CO₂. Le milieu de culture est prélevé (mis à -80°C). Une seconde incubation de 24 heures est effectuée dans les conditions identiques.

La couche cellulaire est incubée en présence de trypsine-EDTA. Les cellules détachées sont centrifugées puis mises en suspension dans 300 µL d'eau distillée. Les IL-1α sont dosées à l'aide du Kit Immunotech dans le compartiment intracellulaire (0-48 heures) et dans le compartiment milieu de culture (0-24 heures et 24-48 heures). Les résultats obtenus sont présentés dans le Tableau 2.

**Tableau 2**

| compartiment intracellulaire (0-48 heures) | | |
|---|---|---|
| µg/mL | IL-1α (pg/puits) | IL-1α (pg/10⁶ cellules) |
| Témoin | 26 ± 7 | 82 ± 22 |
| 500 | 71 ± 24 | 250 ± 83 |
| 100 | 65 ± 9 | 229 ± 31 |
| 50 | 43 ± 5 | 145 ± 17 |
| 10 | 40 ± 8 | 135 ± 27 |
| 1 | 35 ± 3 | 123 ± 9 |
| LPS 1 | 958 ± 85 | 3199 ± 284 |
| LPS 5 | 1651 ± 62 | 5393 ± 201 |

| µg/mL | IL-1α (pg/puits) | IL-1α (pg/10 ⁶ cellules) |
|---|---|---|
| Témoin | 51,6 ± 8,9 | 176 ± 30 |
| 500 + LPS1 | 362,1 ± 91,4 | 1213 ± 306 |
| 100+ LPS1 | 464,1 ± 20,2 | 1543 ± 67 |
| 50 + LPS1 | 506,0 ± 78,5 | 1721 ± 267 |
| 10 + LPS1 | 324,5 ± 87,3 | 1053 ± 283 |
| 1 + LPS1 | 444,8 ± 87,1 | 1389 ± 272 |
| LPS1 | 468,5 ± 122,9 | 1570 ± 412 |

| µg/mL | IL-1α (pg/puits) | IL-1α (pg/10⁶ cellules) |
|---|---|---|
| Témoin | 51,6 ± 8,9 | 175,7 ± 304,4 |
| 500 + LPS 5 | 372,9± 47,3 | 1399,7± 176,2 |
| 100+ LPS 5 | 498.3± 67,9 | 1677,9± 228,6 |
| 50 + LPS 5 | 585.4±64.3 | 1903,7± 209,1 |
| 10 + LPS 5 | 696,0±50,1 | 2188,5± 157,6 |
| 1 + LPS 5 | 1253 ± ----- | 4540 ± ----- |
| LPS 5 | 1208,1 ± 25 | 4216,6 ± 87,2 |

### Résultats

II n'y a pas de variation significative du taux d' IL-1α de base en présence d'un ester de tocophéryle avec un acide gras mono ou polyinsaturé à une concentration de 1 à 500 µg/mL.

Pour une concentration de LPS à 1 et 5 µg/mL en absence d'un ester de tocophéryle avec un acide gras mono ou polyinsaturé, le taux d' IL-1α est respectivement multiplié par 39 et 66, ce qui démontre une bonne réactivité du système test.
- Un ester de tocophéryle avec un acide gras mono ou polyinsaturé à une concentration de 1 à 500 µg/mL n'a pas d'effet significatif sur le taux intracellulaire des IL-1α des monocytes/ macrophages humains activés par des LPS à 1 µg/mL.
- Un ester de tocophéryle avec un acide gras mono ou polyinsaturé exerce une inhibition dose-dépendante maximum pour 500 µg/mL (- 67%) sur le taux intracellulaire des IL-1α des monocytes/ macrophages humains activés par des LPS à 5 µg/mL.

**Tableau 3**

| compartiment milieu de culture (0-24 heures) | |
|---|---|
| µg/mL | IL-1α (pg/puits) |
| Témoin | 99 ± 4 |
| 500 | 157 ± 28 |
| 100 | 94 ± 4 |
| 50 | 116 ± 9 |
| 10 | 107 ± 4 |
| 1 | 103 ± 6 |

### Résultats

Les résultats sont exprimés en pg/puits (il n'y a pas de comptage cellulaire pour ce temps d'incubation).

Il n'y a pas de variation significative du taux d' IL-1α de base en présence d'un ester de tocophéryle avec un acide gras mono ou polyinsaturé à une concentration allant de 1 à 500 µg/mL.

**Tableau 4**

| compartiment milieu de culture (24-48 heures) | | |
|---|---|---|
| µg/mL | IL-1α (pg/puits) | IL-1α (pg/10 ⁶cellules) |
| Témoin | 99 ± 3 | 311 ± 9 |
| 500 | 71 ± 4 | 250 ± 14 |
| 100 | 103 ± 19 | 362 ± 68 |
| 50 | 86 ± 18 | 287 ± 59 |
| 10 | 67 ± 4 | 228 ± 12 |
| 1 | 75 ± 3 | 263 ± 9 |
| LPS1 | 334 ± 35 | 1116 ± 116 |
| LPS 5 | 668 ± 70 | 2181 ± 227 |
| | | |
| Témoin | 35,9 ± 5,3 | 122 ± 18 |
| 500 + LPS1 | 239,6 ± 74,0 | 803 ± 248 |
| 100+LPS1 | 125,5 ± 18,0 | 417 ± 60 |
| 50 + LPS1 | 156,1 ± 23,3 | 531 ± 79 |
| 10 + LPS1 | 124,8 ± 26,9 | 405 ± 87 |
| 1 + LPS1 | 99.5 ± 42,1 | 1389 ± 272 |
| LPS1 | 112., ± 24.3 | 376 ± 82 |
| | | |

| µg/mL | IL-1α (pg/puits) | IL-1α (pg/10⁶ cellules) |
|---|---|---|
| Témoin | 36 ± 5 | 122 ± 18 |
| 500 + LPS 5 | 412 ± 51 | 1534 ± 184 |
| 100+ LPS 5 | 164 ± 21 | 551 ± 71 |
| 50 + LPS 5 | 173 ± 50 | 561 ± 164 |
| 10 +LPS5 | 129 ± 32 | 404 ±10 |
| 1+LPS5 | 178 ± 5 | 647 ±16 |
| LPS 5 | 225 ± 24 | 787 ± *83 |

- Un ester de tocophéryle avec un acide gras mono ou polyinsaturé à une concentration de 1 à 100 µg/ml n'a pas d'effet significatif sur la sécrétion des IL-1α par des monocytes/ macrophages humains activés par des LPS à 1 µg/ml.

Par contre à une concentration de 500 µg/mL en ester de tocophéryle avec un acide gras mono ou polyinsaturé on observe une augmentation (x2) du taux d' IL-1α dans le milieu de culture .

### Conclusion

Un ester de tocophéryle avec un acide gras mono ou polyinsaturé exerce un potentiel de régulation de l'inflammation observé sur la sécrétion des IL-1α par des monocytes/macrophages humains activés par des LPS à 5 µg/mL.

### 3- Etude d'un ester de tocophéryle avec un acide gras mono ou polyinsaturé sur la synthèse de l'élastine et du collagène par des fibroblastes humains.

### a) Préparation des cellules

Les cellules sont ensemencées à la densité de 50 000 cellules/cm² dans des plaques multipuits.

Les cellules sont incubées à 37°C en atmosphère humide contenant 5% (v/v) de CO₂. Le milieu de culture est l'IMDM supplémenté par du sérum de veau foetal (SVF) à 10% (v/v).

Le milieu de culture est remplacé après 24 heures d'incubation et supplémenté par des antibiotiques (Peni-Strepto) à 0,2 % (V/v) contenant les différentes concentrations d'ester de tocophéryle avec un acide gras mono ou polyinsaturé (1, 5, 10,50 et 100 µg/mL).

Le milieu de culture est supplémenté par de l'acide ascorbique à 50 µg/mL.

Une série « témoin positif» est constituée de cellules traitées par le TGF-β à 10 ng/mL.

### b) Evaluation de la synthèse de l'élastine et du collagène

Les cellules sont incubées à 37°C en atmosphère humide contenant 5% (v/v) de CO₂ pendant 24 heures.

A la fin de la période d'incubation, le milieu de culture est prélevé et la couche cellulaire rincée deux fois avec du PBS supplémenté d'inhibiteurs de protéases afin d'obtenir les concentrations suivantes : EDTA 0,01 M, acide 6-amino hexanoïque 0,1 M, chlorure de benzaminidium 0,005 M, iodoacétamide 0,1mM.

La couche cellulaire est grattée dans 500 µL de PBS contenant les inhibiteurs de protéases puis est passée aux ultrasons (2x20 sec à intervalles de 20 sec ; 50Hz)

Le dosage colorimétrique de l'élastine est réalisé à partir de la liaison spécifique du colorant 5,10,15,20 tétraphényl-21, 23 porphine sulphate (TPPS) (Kit fastin Elastin Assay)

### Résultats

Matrice extracellulaire

**Tableau 5**

| µg/mL | Elastine (µg/puits) | Elastine (µg/10⁶ cellules) |
|---|---|---|
| Témoin | 38,5 ± 4,7 | 289 ± 35 |
| TGF-β à 10 ng/mL | 56,4 ± 2,3 | 422 ± 17 |
| 1 | 31,7 ± 9,3 | 242 ± 71 |
| 5 | 23,2 ± 1,7 | 174 ± 13 |
| 10 | 31,8 ± 4,5 | 241 ± 34 |
| 50 | 32,2 ± 2,0 | 260 ± 16 |
| 100 | 16,4 ± 0,9 | 139 ± 8 |

**Tableau 6**

| µg/mL | Collagène (µg/puits) | Collagène(µg/10⁶ cellules) |
|---|---|---|
| Témoin | 37,5 ± 4.4 | 281 ± 33 |
| TGF-β à 10 ng/mL | 76,9 ± 13,0 | 576 ± 97 |
| 1 | 30,2 ± 15,5 | 230 ± 118 |
| 5 | 29,8 ± 10,3 | 224 ±77 |
| 10 | 43,4 ±6,5 | 329 ±49 |
| 50 | 50,4 ± 15,9 | 407 ± 129 |
| 100 | 39,4 ± 8,6 | 336 ± 73 |

Dans le milieu de culture

**Tableau 7**

| µg/mL | Elastine (µg/puits) | Elastine (µg/10⁶ cellules) |
|---|---|---|
| Témoin | 77,4 ± 3,7 | 580 ± 28 |
| TGF-β à 10 ng/mL | 83,3 ± 0,1 | 624 ± 1 |
| 1 | 68,2± 11,2 | 521 ± 85 |
| 5 | 78,2 ±16.5 | 587 ± 124 |
| 10 | 150,5 ± 12,4 | 1140 ± 94 |
| 50 | 145,5 ± 5,8 | 1176 ± 47 |
| 100 | 121,9 ± 5,5 | 1037 ± 47 |

**Tableau 8**

| µg/mL | Collagène (µg/puits) | Collagène (µg/10⁶ cellules) |
|---|---|---|
| Témoin | 47 ± 12 | 355 ± 91 |
| TGF-β à 10 ng/mL | 65 ± 10 | 484 ± 75 |
| 1 | 39 ± 12 | 297 ± 95 |
| 5 | 66 ± 26 | 499 ± 196 |
| 10 | 72 ± 40 | 542 ± 306 |
| 50 | 66 ± 19 | 533 ± 150 |
| 100 | 48 ± 29 | 409 ± 249 |

Les concentrations entre 10 et 100 µg/ml d'un ester de tocophéryle avec un acide gras mono ou polyinsaturé stimulent significativement la synthèse de l'élastine et du collagène. L'effet est maximum pour 50 µg/mL (+ 65% pour l'élastine et + 45% pour le collagène) avec une augmentation de la sécrétion de l'élastine dans le milieu de culture (+ 102%), alors que l'effet du TGF-β à 10 ng/mL se traduit principalement par une augmentation de la synthèse au niveau de la matrice extracellulaire (+ 46%).

### 4- Etude d'un ester de tocophéryle avec un acide gras mono ou polyinsaturé sur l'action anti UVB.

### a) Préparation des feuillets épidermiques

Les kératinocytes humains sont ensemencées à la densité de 100 000 cellules/cm² dans des inserts dont le fond est constitué d'une membrane en polycarbonate de porosité 0,4 µm.

Les cellules sont incubées à 37°C en atmosphère humide contenant 5% (v/v) de CO₂ pendant 24 à 48 heures, en « situation immergée » dans le milieu de culture MCDB/153 supplémenté par l'EGF (5 ng/mL), l'insuline (5 µg/ml), l'hydrocortisone (5 ng/mL), le BPE (70 µg/mL)

Le milieu de culture est remplacé par le même milieu de culture supplémenté par 1 mM de Ca⁺⁺ afin d'induire la stratification de la monocouche en « situation immergée » pendant 6 jours. Le milieu de culture est changé tous les 3 jours .

Le feuillet épidermique est mis en « situation émergée » au 7èmr jour, c'est à dire qu'il n'est plus nourri que par l'intermédiaire des cellules basales.

Au 14^{ème} jour d'incubation, le milieu de culture est changé. Différentes concentrations d'un ester de tocophéryle avec un acide gras mono ou polyinsaturé ( 1, 5, 10, 50 et 100 µg/mL), ou de vitamine E ( 150 µl) constituant le « témoin positif » sont appliquées sur un disque de papier Whatman couvrant la surface de la culture cellulaire.

### b) Evaluation de la cytotoxicité ( Test au MTT)

Les cellules sont incubées à 37°C en atmosphère humide contenant 5% (v/v) de CO₂ pendant 24 heures, puis irradiées par les UVB (0,7 J/cm², λ= 314 nm) après avoir enlevé les disques de papier Whatman.

Les cellules sont incubées à 37°C en atmosphère humide contenant 5% (v/v) de CO₂ pendant 24 heures, après avoir déposé sur la surface de la culture cellulaire les disques de papier Whatman imbibés des différentes concentrations d'un ester de tocophéryle avec un acide gras mono ou polyinsaturé ou de vitamine E.

Après l'incubation, les milieux de culture sont utilisés pour le dosage des produits du clivage du MTT.

Après l'incubation, les membranes portant le feuillet épidermique sont rincées 2 fois avec du PBS, puis incubée 3 heures à 37 °C , à l'obscurité, en présence de 2 mL de MTT (bromure de 3( 4,5-diméthylthiazol-2-yl)-2,5 diphényl tétrazolium) à 0,5 mg/mL. Les cristaux violets de formazan produits par le clivage du MTT par les enzymes mitochondriaux sont dissous dans 2 mL de DMSO sous agitation douce pendant 2 heures à température ambiante.

On mesure l'absorption à 540 nm au spectrophotomètre.

**Tableau 9**

| compartiment intracellulaire | | | | | | | |
|---|---|---|---|---|---|---|---|
| | témoin | témoin + UVB | 1 µg/mL | 5 µg/mL | 10 µg/mL | 50 µg/mL | 100 µg/mL |
| moyenne absorbance à 540 nm | 1,504 ± 0.084 | 0.710 ± 0.076 | 0.693 ± 0.068 | 0.682 ± 0.069 | 1,049 ± 0.117 | 0,698 ± 0.035 | 0,809 ± 0.132 |

**Tableau 10**

| compartiment milieu de culture | | | | | | | |
|---|---|---|---|---|---|---|---|
| | témoin | témoin + UVB | 1 µg/mL | 5 µg/mL | 10 µg/mL | 50 µg/mL | 100 µg/mL |
| moyenne absorbance à 540 nm | 1,504 ± 0.084 | 0.710 ± 0.076 | 0.693 ± 0.068 | 0.682 ± 0.069 | 1,049 ± 0.117 | 0,698 ± 0.035 | 0,809 ± 0.132 |

On observe une inhibition sensible de l'effet cytotoxique induit par l'irradiation, en présence d'une concentration en ester d'acide gras de tocophérol à 10 µg/ml.

### 5- Etude d'un ester de tocophéryle avec un acide gras mono ou polyinsaturé sur l'activité mitochondriale de fibroblastes de derme humain.

### a) Préparation des cellules

Les fibroblastes de derme humain sont ensemencées à la densité de 5000 cellules/cm² dans 500 µL de milieu IMDM supplémenté par du SVF 10% pendant 24 heures en atmosphère humide contenant 5% (v/v) de CO₂

Après 24 heures, les cellules sont mises en contact avec les différentes concentrations d'un ester de tocophéryle avec un acide gras mono ou polyinsaturé ( 10, 50 et 100 µg/mL).

Ce sont des concentrations non cytotoxiques qui ne modifient pas la croissance cellulaire.

Une série « témoin positif» est constituée de cellules traitées à l'antimycine à 0,5 µM.

### b) Evaluation de l'activité mitochondriale

Principe : L'incorporation cellulaire de rhodamine 123 est la conséquence de la variation du potentiel transmembranaire mitochondrial. L'évaluation semi-quantitative de la respiration mitochondriale est basée sur l'intensité de la fluorescence à l'intérieur de la cellule.

Les cellules sont incubées pendant 72 heures à 37°C en atmosphère humide contenant 5% (v/v) de CO₂.

A la fin de la période d'incubation, le milieu de culture est prélevé et la couche cellulaire rincée deux fois avec du milieu IMDM puis mise dans 500 µL de tampon phosphate pH 7.2 contenant 0.1 mg/mL de rhodamine 123.

On laisse incuber pendant 15 minutes à 37 °C, puis on mesure la fluorescence de façon semi-quantitative à l'aide d'une échelle de cotation.

### Résultats

**Tableau 11**

| µg/mL | 4 | 3 | 2 | 1 | 0 | Nombre cellules observées |
|---|---|---|---|---|---|---|
| Témoin | 12,3 ± 2,1 | 25,7 ± 3,1 | 25,0 ± 2,4 | 21,0 ± 2,9 | 17,7 ± 2,1 | 104,0 ± 9 |
| Témoin Antimycine | 10,7 ± 2,5 | 13,3 ± 2,4 | 20,0 ± 1,6 | 16,3 ± 1,9 | 25,0 ± 4,1 | 100 ± 9 |
| 10 | 19,7 ± 1,2 | 32,3 ± 2,1 | 26,3 ± 2,4 | 14,7 ± 0,5 | 8,7 ± 1,9 | 102,0 ± 5 |
| 50 | 35,3 ± 0,5 | 44,0 ± 1,4 | 18,3 ± 8,5 | 3,3 ± 0,5 | 0,0 ± 0,0 | 102,0 ± 9 |
| 100 | 12,3 ± 2,1 | 58,3 ± 6,2 | 20,3 ± 0,5 | 9,3 ± 0,5 | 0,0 ± 0,9 | 100,0 ± 9 |

Echelle de cotation (Intensité de fluorescence) : 0 : cytoplasme non fluorescent, 1 : faible ; 2 : moyennement intense ; 3 : Importante ; 4 : Très importante, tout le cytoplasme est fluorescent.

La stimulation est maximale pour une concentration d'un ester de tocophéryle avec un acide gras mono ou polyinsaturé à 50 µg/ml ; +65% par rapport au témoin.

## Revendications

1. Utilisation d'esters de tocophérol répondant à la formule générale I 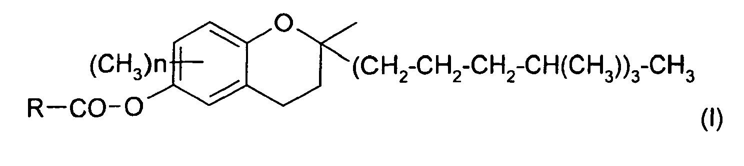 dans laquelle :
- n est un nombre entier variant de 1 à 3
- R est le reste de la chaîne aliphatique d'un acide gras monoinsaturé ayant de 11 à 22 atomes de carbone, ou d'un acide gras polyinsaturé choisi parmi les acides α-linolénique (C18:3 n-3) , octadécatétraénoïque (C18:4 n-3), eicosatétraénoïque (C20:4 n-3), docosahexaénoïque (C22:6 n-3), linoléique (C18:2 n-6), γ-linolénique (C18:3 n-6), dihomo- γ-linolénique (C20:3 n-6), arachidonique (C20:4 n-6), et docosapentaénoïque (C22:5 n-6)
en vue de la réalisation de compositions pharmaceutiques, cosmétiques et/ou diététiques destinées au traitement réparateur de la peau par action sur la synthèse des constituants du derme et présentant une action stimulante sur la capacité respiratoire des mitochondries.

2. Utilisation d'esters de tocophérol selon la revendication 1, en vue de la réalisation de compositions pharmaceutiques, cosmétiques et/ou diététiques **caractérisées en ce que** l'action stimulante s'exerce sur des mitochondries de fibroblastes de derme humain.

3. Utilisation d'esters de tocophérol selon la revendication 1 ou la revendication 2, en vue de la réalisation de compositions pharmaceutiques, cosmétiques et/ou diététiques présentant en outre une action anti-UVB.

4. Utilisation d'esters de tocophérol selon l'une des revendications précédentes, en vue de la réalisation de compositions pharmaceutiques, cosmétiques et/ou diététiques présentant en outre une action stimulante sur la tyrosine kinase.

5. Utilisation d'esters de tocophérol selon l'une des revendications précédentes, en vue de la réalisation de compositions pharmaceutiques, cosmétiques et/ou diététiques présentant en outre une action anti-inflammatoire.

6. Utilisation d'esters de tocophérol selon l'une des revendications précédentes, pour stimuler la synthèse du collagène et de l'élastine.

7. Utilisation des esters de formule I selon l'une des revendications précédentes, **caractérisée en ce que** les esters d'acides gras monoinsaturés sont choisis dans le groupe formé des esters d'acide undécylénique (C11 :1) et des esters d'acide palmitoléique (C16 : 1)

8. Utilisation d'esters de tocophérol de formule I selon l'une des revendications précédentes, **caractérisée en ce que** le tocophérol est choisi parmi l' α-tocophérol, le β-tocophérol, le γ-tocophérol et le δ-tocophérol.

9. Utilisation d'au moins un ester de tocophérol selon l'une des revendications précédentes, avec des excipients ou des véhicules appropriés pour la réalisation de compositions pharmaceutique, cosmétique et/ou diététique utiles en application topique chez l'homme ou l'animal.

10. Utilisation d'esters de formule I selon l'une des revendications précédentes, **caractérisée en ce que** la teneur en ester de formule I dans une composition pharmaceutique, cosmétique et/ou diététique varie de 0,001 % à 15 %, en fonction du mode d'administration ou d'application desdites compositions.

11. Utilisation d'esters de formule I selon l'une des revendications précédentes, **caractérisée en ce que** la teneur en ester de formule I dans une composition pharmaceutique, cosmétique et/ou diététique varie de 0,01 à 5%, en fonction du mode d'administration ou d'application desdites compositions.

## Claims

1. Use of tocopherol esters having the general formula I: 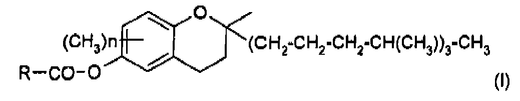 wherein:
- n is an integer varying from 1 to 3,
- R is the rest of the aliphatic chain of a monounsaturated fatty acid having between 11 and 22 carbon atoms, or a polyunsaturated fatty acid selected from among the following acids α-linolenic (C18:3 n-3), octadecatetraenoic (C18:4 n-3), eicosatetraenoic (C20:4 n-3), docosahexaenoic (C22:6 n-3), linoleic (C18:2 n-6), γ-linolenic (C18:3 n-6), dihomo-γ-linolenic (C20:3 n-6), arachidonic (C20:4 n-6) and docosapentaenoic (C22:5 n-6), with a view to the manufacture of pharmaceutical, cosmetic and/or dietetic compositions intended for the reparative treatment of the skin by action on the synthesis of the dermal constituents and having a stimulating action on the respiratory capacity of the mitochondria.

2. Use of tocopherol esters according to Claim 1, with a view to manufacturing pharmaceutical, cosmetic and/or dietetic compositions **characterised in that** the stimulant action is exerted on the mitochondria of human dermal fibroblasts.

3. Use of tocopherol esters according to Claim 1 or Claim 2, with a view to manufacturing pharmaceutical, cosmetic and/or dietetic compositions additionally having an anti-UVB action.

4. Use of tocopherol esters according to one of the above Claims with a view to manufacturing pharmaceutical, cosmetic and/or dietetic compositions additionally having a stimulant action on tyrosine kinase.

5. Use of tocopherol esters according to one of the above Claims with a view to manufacturing pharmaceutical, cosmetic and/or dietetic compositions additionally having an antiinflammatory action.

6. Use of tocopherol esters according to one of the above Claims to stimulate the synthesis of collagen and elastin.

7. Use of formula I esters according to one of the above Claims **characterised in that** the monounsaturated fatty acid esters are selected from the group formed of the undecylenic acid esters (C11:1) and the palmitoleic acid esters (C16:1).

8. Use of formula I tocopherol esters according to one of the above Claims **characterised in that** the tocopherol is selected from among α- tocopherol, β- tocopherol, γ-tocopherol or 5- tocopherol.

9. Use of at least one tocopherol ester according to one of the above Claims with excipients or vehicles suitable for the manufacture of pharmaceutical, cosmetic and/or dietetic compositions useful for topical application on a human or animal.

10. Use of formula I esters according to one of the above Claims, **characterised in that** the potency of the formula I ester in a pharmaceutical, cosmetic and/or dietetic composition varies from 0.001% to 15% depending on the delivery or application system employed for said compositions.

11. Use of formula I esters according to one of the above Claims, **characterised in that** the potency of the formula I ester in a pharmaceutical, cosmetic and/or dietetic composition varies from 0.01 to 5% depending on the delivery or application system employed for said compositions.

## Patentansprüche

1. Verwendung von Tocopherolestern, die der allgemeinen Formel I 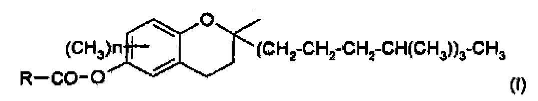 entsprechen, in welcher:
- n eine variable ganze Zahl zwischen 1 und 3 ist
- R der Rest der aliphatischen Kette einer einfach ungesättigten Fettsäure mit 11 bis 22 Kohlenstoffatomen ist, oder einer mehrfach ungesättigten Fettsäure, die aus der α-Linolensäure (C18:3 n-3), Octadecatriensäure (C18:4 n-3), Eicosatriensäure (C20:4 n-3), Docosahexaensäure (C22:6 n-3), Linolsäure (C18:2 n-6), γ-Linolsäure (C18:3 n-6) Dihomo γ-Linolensäure (C20:3 n-6), Arachidonsäure (C20:4 n-6) und Docosapentensäure (C22:5 n-6) ausgewählt wird,
in Hinsicht auf die Herstellung von pharmazeutischen, kosmetischen und / oder diätetischen Zusammensetzungen, die zur stärkenden Behandlung der Haut durch Einwirkung auf die Synthese der Bestandteile der Lederhaut vorgesehen sind und die eine stimulierende Wirkung auf die atmenden Fähigkeiten der Mitochondrien aufweisen.

2. Verwendung von Tocopherolestern nach Anspruch 1 in Hinsicht auf die Herstellung von pharmazeutischen, kosmetischen und/oder diätetischen Zusammensetzungen, **dadurch gekennzeichnet, dass** sich die stimulierende Wirkung auf die Mitochondrien von Fibroblasten menschlicher Lederhaut entfaltet.

3. Verwendung von Tocopherolestern nach Anspruch 1 oder Anspruch 2 in Hinsicht auf die Herstellung von pharmazeutischen, kosmetischen und/oder diätetischen Zusammensetzungen, die außerdem eine Anti-UVB Wirkung aufweisen.

4. Verwendung von Tocopherolestern nach einem der vorhergehenden Ansprüche in Hinsicht auf die Herstellung von pharmazeutischen, kosmetischen und / oder diätetischen Zusammensetzungen, die eine außerdem stimulierende Wirkung auf die Tyrosinkinase aufweisen.

5. Verwendung von Tocopherolestern nach einem der vorhergehenden Ansprüche in Hinsicht auf die Herstellung von pharmazeutischen, kosmetischen und / oder diätetischen Zusammensetzungen, die außerdem eine entzündungshemmende Wirkung aufweisen.

6. Verwendung von Tocopherolestern nach einem der vorhergehenden Ansprüche, um die Synthese des Kollagen und des Elastin zu stimulieren.

7. Verwendung der Ester von Formel I nach einem der vorhergehenden Ansprüche, **dadurch gekennzeichnet, dass** die Ester der einfach ungesättigten Fettsäuren aus der Gruppe ausgewählt werden, die von undezylenischen Fettestern (C11 :1) und der Palmitoleinsäureester (C16 :1) gebildet wird.

8. Verwendung von Tocopherolestern von Formel I nach einem der vorhergehenden Ansprüche, **dadurch gekennzeichnet, dass** das Tocopherol aus dem α-Tocopherol, dem β-Tocopherol, dem γ-Tocopherol und dem δ-Tocopherol ausgewählt wird.

9. Verwendung von wenigstens einem Tocopherolester nach einem der vorhergehenden Ansprüche mit geeigneten Grundmassen oder Stoffträgern für die Herstellung von pharmazeutischen, kosmetischen und / oder diätetischen Zusammensetzungen, die nützlich für topische Anwendungen beim Menschen oder Tier sind.

10. Verwendung von Estern von Formel I nach einem der vorhergehenden Ansprüche, **dadurch gekennzeichnet, dass** der Gehalt an Ester von Formel I in einer pharmazeutischen, kosmetischen und/oder diätetischen Zusammensetzung in Abhängigkeit von der Art der Verabreichung oder der Anwendung der genannten Zusammensetzungen zwischen 0,001% und 15% variiert.

11. Verwendung von Estern von Formel I nach einem der vorhergehenden Ansprüche, **dadurch gekennzeichnet, dass** der Gehalt an Ester von Formel I in einer pharmazeutischen, kosmetischen und/oder diätetischen Zusammensetzung in Abhängigkeit von der Art der Verabreichung oder der Anwendung der genannten Zusammensetzungen zwischen 0,01% und 5% variiert.
